# EUROPÄISCHE PATENTSCHRIFT

(11) **EP 2 402 073 B1**
(45) Veröffentlichungstag und Bekanntmachung des Hinweises auf die Patenterteilung: **23.01.2013**
(21) Anmeldenummer: 11007778.1
(22) Anmeldetag: 27.03.2009
(51) Int. Cl.: B01F 15/00, B01F 7/00, B01F 7/22, C12M 1/00, C12M 1/02, F16C 3/03

(54) **Flexibler Beutel mit einer Mischvorrichtung**
Flexible bag with a mixing device
Sachet souple doté d'un dispositif de mélange

(30) Priorität: 17.04.2008 DE 102008019213
(43) Veröffentlichungstag der Anmeldung: 04.01.2012
(62) Teilanmeldung aus: 09753580.1
(73) Patentinhaber: Sartorius Stedim Biotech GmbH, 37079 Göttingen (DE)
(72) Erfinder: Pradel, Günter, 37077 Göttingen (DE); Greller, Gerhard, Dr., 37085 Göttingen (DE)

(56) Entgegenhaltungen:
- EP-A- 1 541 224
- DE-A1-102006 021 984
- GB-A- 1 344 316
- US-A- 3 246 882
- US-A- 5 765 947
- US-A1- 2007 102 450

## Beschreibung

Die Erfindung betrifft einen flexiblen Beutel mit einer Mischvorrichtung, deren Mischerschaft in seiner Länge verstellbar ist, insbesondere für die Verwendung als Bioreaktor zur Kultivierung von Mikroorganismen und Zellen oder als Mischvorrichtung für Medien oder zum Lösen oder Suspendieren von Stoffen in Flüssigkeiten, wie etwa zur Herstellung von Pufferlösungen.

Bioreaktoren, in denen das Kulturmedium in flexiblen Beuteln aus Polymermaterial aufgenommen wird, sind für einen Volumenbereich erhältlich, der sich von wenigen Litern bis hin zu mehreren hundert Litern erstreckt. Besonders bei Beuteln mit großen Volumina erweist sich die Durchmischung des Kulturmediums, durch die ein einheitlicher Sauerstoffpartialdruck gewährleistet werden soll, als problematisch. Zum Zweck der Durchmischung kommen sehr häufig rotierende Rührstäbe zum Einsatz, die aus einem Mischerschaft und einem oder mehreren Rührelementen, wie z.B. Propellern, bestehen und die in den Innenraum des Beutels eingebracht werden. Daneben gibt es auch Rührelemente in Form von Paddeln oder vertikal vibrierenden Mischerplatten. Da bei einem Einwegbioreaktor idealerweise sämtliche Teile, die während der Kultivierung mit Zellen in Kontakt kommen, entsorgt werden können, ist es von Vorteil, auch den Mischerschaft aus günstigem Plastikmaterial herzustellen.

Hinsichtlich der Lagerung und des Transports von Einwegbioreaktoren mit einem Volumen von 200 bis 1000 Litern stellt der Mischerschaft im Gegensatz zum Beutel, der gefaltet und komprimiert werden kann, ein Problem dar, da seine Länge leicht die Größe der gängigen Verpackungs- und Lagereinheiten übertrifft.

Auch nach der Kultivierung kann es von Vorteil sein, den Beutel wieder auf ein kleineres Format zu bringen, z.B. wenn das zellhaltige Kulturmedium gekühlt und gelagert werden soll. In diesem Fall könnte das im Kopfraum des Beutels befindliche Gas abgelassen werden, wodurch die Höhe des Beutels in der Regel um ein Drittel reduziert werden könnte, was jedoch einen längenverstellbaren Mischerschaft voraussetzen würde. Ein ähnlich gelagertes Problem tritt auf, wenn die im Beutel enthaltenen Zellen oder Mikroorganismen nach einer fehlgeschlagenen Kultivierung durch Hitze abgetötet werden sollen, und der Mischerschaft sich für den Autoklaven als zu sperrig erweist.

Aus der deutschen Patentanmeldung DE 10 2006 021 984 A1, die den Oberbegriff des Anspruch 1 offenbart ist es bekannt, einen Mischerschaft aus mehreren separaten Teilen zusammen zu setzen, mit dem Ziel, Bioreaktoren verschiedener Größe mit einheitlichen Mischerbauteilen ausstatten zu können. Grundsätzlich wäre es auch für die Verringerung der Lager- und Transporthöhe des Beutels möglich, den Mischerschaft in mehreren Einzelteilen zu liefern, wie in DE 10 2006 021 984 A1 beschrieben, und die Mischvorrichtung erst vor der Inbetriebnahme zu montieren. Dieses Vorgehen scheitert jedoch daran, dass sich die separaten Mischerschaftteile bei der Anlieferung bereits vorsterilisiert im Innenraum des sterilen Beutels befinden und deshalb kaum zum Mischerschaft zusammen gesetzt werden könnten, ohne den sterilen Innenraum des Beutels zu öffnen.

JP 06 285353 A beschreibt einen Behälter mit flexibler Behälterwandung und einem Behälterinnenraum, in dem an einem Ende eines durch die Behälterwandung hindurchgeführten, von außen antreibbaren hohlen Mischerschaftes ein Mischer angeordnet ist, wobei mindestens ein Teil des in den Behälterinnenraum ragenden Mischerschaftes faltbar ist und durch von außen über eine Luftpumpe einbringbare Luft entfaltet wird.

GB 1 344 316 A offenbart einen Mischbehälter mit einer Mischvorrichtung, welche einen längenverstellbaren, teleskopartigen Mischerschaft aufweist, der in zwei Schaftelemente unterteilt ist. Dabei weist das erste der beiden benachbarten Schaftelemente einen Hohlkörper auf, in dem ein Füllkörper des zweiten Schaftelements axial verschiebbar aufgenommen ist, wobei sich in dem Spalt zwischen dem Hohlkörper des ersten Schaftelements und dem Füllkörper des zweiten Schaftelements ein elastisches Dichtungselement befindet.

Die Aufgabe der Erfindung besteht deshalb darin, einen flexiblen Beutel mit einer Mischvorrichtung vorzuschlagen, deren Mischerschaft auf einfache Art und Weise im sterilen Innenraum des Beutels von einer verkürzten Transport-/Lagerlänge in eine verlängerte Betriebslänge überführbar ist, ohne dass dabei die sterile Hülle des Bioreaktors geöffnet werden muss, bzw. der wieder auf seine ursprüngliche Transport-/Lagerlänge reduzierbar ist, ohne dass dabei der Beutel geöffnet werden müsste, und bei welchem beim Ausziehen des Schaftes ein Druckausgleich stattfindet, ohne dass sich im Innern des Mischerschafts ein Unterdruck ausbildet und ohne dass die Gefahr einer Kontamination des Beutelinnenraums besteht.

Die Aufgabe wird erfindungsgemäß durch den Gegenstand des Anspruchs 1 gelöst. Die Erfindung umfasst einen flexiblen Beutel mit einer Mischvorrichtung, die einen längenverstellbaren Mischerschaft aufweist, der in mindestens zwei oder mehrere Schaftelemente unterteilt ist, wobei ein erstes von zwei benachbarten Schaftelementen einen Hohlkörper aufweist, in dem ein Füllkörper des zweiten Schaftelements verschiebbar aufgenommen ist, wobei sich in dem Spalt zwischen dem Hohlkörper des ersten Schaftelements und dem Füllkörper des zweiten Schaftelements mindestens ein elastisches Element befindet und wobei ein Hohlraum im Innenraum des Mischerschaftes mit dem Innenraum des Beutels zum Druckausgleich durch einen Kanal verbunden ist.

Zumindest die beiden äußeren Schaftelemente sind dabei auf der dem Beutel zugewandten Seite geschlossen.

Die Hohlkörper eines ersten von zwei benachbarten Schaftelementen und der Füllkörper des zweiten Schaftelements weisen eine gemeinsame, geschlossen umlaufende Kontur auf, wobei drehrunde, ovale oder vieleckige Konturen bevorzugt sind. Das elastische Element, welches sich im Spalt zwischen dem Hohlkörper des ersten Schaftelements und dem Füllkörper des zweiten Schaftelements befindet, besteht bevorzugt aus zwei beabstandeten Ringen, die beispielsweise aus einem Kunststoff, wie Gummi, Silikon gefertigt sein können. Zur Befestigung können die beiden beabstandeten Ringe teilweise in eine Nut eingelassen sein, die sich auf dem Füllkörper des zweiten Schaftelements oder in dem Hohlkörper des ersten Schaftelements befindet, oder durch Mehr-, insbesondere 2-K-Technologie (Mehr- und 2-K stehen für Mehrkomponenten- und zwei Komponenten in der Spritzgusstechnologie) an den Füllkörper des zweiten Schaftelements oder an den Hohlkörper des ersten Schaftelements angeformt sein. Die Ringe übernehmen zwei wichtige Funktionen für die Verbindung des Hohlkörpers mit dem Füllkörper: Zum einen gewährleisten sie durch einen engen Kontakt mit dem Füllkörper und mit dem Hohlkörper, dass das Drehmoment des rotierenden Mischerschaftes zwischen dem Hohlkörper und dem Füllkörper und somit zwischen den beiden Schaftelementen übertragen wird. Zum zweiten wird der Hohlkörper des ersten Schaftelements durch die Ringe gegen Flüssigkeiten aus dem Innenraum des Beutels abgedichtet. Dies ist besonders wichtig, da sich im Mischerschaft bei ausgezogenem Füllkörper ein Hohlraum ausbildet, der sich ohne Dichtung mit Kulturmedium und absterbenden Zellen anfüllen würde, was sich auf die Gesamtkultur negativ auswirken könnte.

Der Einsatz des elastischen Elements, z.B. in Form von Ringen, hat außerdem den erheblichen Vorteil, dass die Arretierung einer gewünschten Länge des Mischerschaftes allein schon durch die zwischen dem Hohlkörper, dem elastischen Element und dem Füllkörper wirkende Reibungskraft gewährleistet ist. Auf andere Arretierungsmechanismen, in etwa Kugelrasten, die metallische Bauteile aufweisen, kann beim Einsatz von elastischen Elementen deshalb verzichtet werden. Dies bringt Vorteile mit sich, wenn der Beutel mit Gammastrahlen sterilisiert werden soll, da die metallischen Bauteile Keime von den Strahlen abschirmen könnten.

Eine weitere Funktion des mindestens einen elastischen Elements besteht darin, den Hohlraum im Inneren des Mischerschaftes gegen Flüssigkeiten aus dem Innenraum des Beutels abzudichten, wobei der Hohlraum im Inneren des Mischerschaftes mit dem Innenraum des Beutels durch den erfindungsgemäss vorgesehenen Kanal in Verbindung steht, der einen Druckausgleich zulässt, welcher für den Druckausgleich den Hohlraum im Inneren des Mischerschaftes mit dem gasgefüllten Kopfraum des Beutels verbindet. In einer abgewandelten Ausführung kann der Kanal gegen Flüssigkeiten durch eine mikroporöse, hydrophobe Membran abgedichtet sein.

In einer weiteren Ausführungsform kann jedes der Schaftelemente mit mindestens einem Rührelement bestückt sein.

Zum Arretieren des Mischerschaftes in einer bestimmten Stellung kann der Hohlkörper und/oder der Füllkörper eine Nut aufweisen, in die eines der in einer Nut sitzenden bzw. durch 2-K.-Technologie angeformten elastischen Elemente einrastbar ist.

Prinzipiell sind viele Varianten denkbar, eine Längenverstellung des Mischerschaftes in einem Bioreaktor durchzuführen. Unter der Voraussetzung, dass der Mischerschaft nur indirekt über die relativ rigide Hülle des Beutels manipuliert werden kann (wie in der Aufgabenstellung formuliert), hat es sich als besonders vorteilhaft erwiesen, die Verbindung der Schaftelemente ausziehbar zu gestalten, wobei das erste von zwei benachbarten Schaftelementen einen Hohlkörper aufweist, in dem der Füllkörper des zweiten Schaftelements longitudinal verschiebbar aufgenommen ist. Die Längenverstellung ist insbesondere dann einfach durchführbar, wenn der Mischerschaft durchgehend ausgeführt ist, d.h. wenn das erste Ende des Mischerschaftes oben und das zweite Ende unten im Beutel gelagert ist, womit die Enden als Angriffspunke für die Längenverstellung dienen können.

Der Querschnitt des Hohlköpers und des Füllkörpers kann grundsätzlich eine Vielzahl von Formen annehmen, wobei drehrunde Hohlkörper und Füllkörper den Vorteil haben, dass sie leicht zu fertigen sind, und eine Abdichtung des Hohlkörpers des ersten Schaftelements durch runde elastische Elemente erheblich einfacher ist als bei nicht-drehrunden Ausführungen.

Im Falle einer drehrunden Ausführung des Hohlkörpers und des Füllkörpers überträgt das mindestens eine elastische Element bei der Rotation des Mischerschaftes ein Drehmoment zwischen dem Hohlkörper des ersten Schaftelements und dem Füllkörper des zweiten Schaftelements, wobei das übertragene Drehmoment bevorzugt 0 bis 50 Nm beträgt.

Nicht drehrunde Hohlköper und Füllkörper haben dagegen den Vorteil, dass das beim Rühren auftretende Drehmoment schon aufgrund der Verrastung der Formen zwischen den beiden Schaftelementen übertragbar ist.

Die Befestigung des elastischen Elements erfolgt bevorzugt, indem eine Nut in den Füllkörper oder in den Hohlkörper eingebracht wird, in welcher das elastische Element teilweise eingelassen wird. Alternativ kann das elastische Element auch durch 2-K-Technologie an den Füllkörper bzw. den Hohlkörper angebracht werden.

Um eine bestimmte Länge des Mischerschaftes zu arretieren, kann einer der Ringe, der sich auf dem Füllkörper befindet, in eine Nut, die in die Innenseite des Hohlkörpers eingearbeitet ist, einrasten.

Die Verwendung mindestens eines elastischen Elements zwischen dem ersten und dem zweiten Schaftelement ist gerade für drehrunde Hohlkörper und Füllkörper eine bevorzugte Lösung, da das Drehmoment des Rührstabes, das zum Durchmischen eines Einwegbioreaktor aufgewendet werden muss, in der Regel nicht sehr groß ist. Dies bedeutet, dass die für das Rühren zwischen den Ringen und den beiden Schaftelementen wirkende Reibungskraft ebenfalls nicht sehr groß sein muss. Stellt man die Reibungskraft über den Anpressdruck der Ringe gerade so groß ein, dass das zum Rühren maximal notwendige Drehmoment übertragen werden kann, so ist auch die Reibungskraft, die bei dem longitudinalen Verschieben während der Längenverstellung auftritt, relativ gering und kann in der Regel manuell überwunden werden (vgl. Ausführungsbeispiel 1).

Mit anderen Worten, Ringe mit passend ausgewähltem Anpressdruck erlauben zum einen das für die Längenverstellung notwendige, longitudinale Verschieben der Schaftelemente unter moderatem Kraftaufwand und verhindern zum anderen jedoch ein radiales Gleiten der Ringe auf dem Hohlkörper oder dem Füllkörper beim Rühren.

Darüber hinaus übernehmen die Ringe auch eine wichtige Dichtungsfunktion, indem sie verhindern, dass Kulturmedium aus dem Innenraum des Beutels in einen Hohlraum des Mischerschaftes eindringt. Dieser Hohlraum ist zumindest dann vorhanden, wenn ein ausgezogener Mischerschaft vorliegt. In diesem Fall umfasst der Hohlraum zumindest einen Teil des Hohlkörpers eines ersten von zwei benachbarten Schaftelementen, aus dem der Füllkörper des zweiten Schaftelements teilweise herausgezogen wurde. Der Hohlraum im Inneren des Mischerschaftes kann sich auch über mehrere Schaftelemente erstrecken, z.B. wenn der Füllkörper des zweiten von zwei benachbarten Schaftelementen seinerseits hohl ausgebildet ist. Durch die Abdichtung wird verhindert, dass sich im Hohlraum im Inneren des Mischerschaftes ein mit Kulturmedium und absterbenden Zellen gefülltes Totvolumen ausbildet, was sich negativ auf die gesamte Kultur auswirken könnte.

Aufgrund der Abdichtung des Hohlraums im Inneren des Mischerschaftes durch die Ringe oder ein anders geartetes elastische Element muss allerdings dafür gesorgt werden, dass beim Ausziehen des Schaftes ein Druckausgleich statt findet, da sich andernfalls im Innern des Mischerschaftes ein Unterdruck ausbilden würde, der die ausgezogenen Schaftelemente in ihre Ausgangsposition zurück zwingen würde. Dieser Druckausgleich wird erfindungsgemäß durch in den Hohlraum des Mischerschaftes nachströmendes Gas gewährleistet. Die Zufuhr dieses Gases erfolgt über den erfindungsgemäss vorgesehenen Kanal, der den Hohlraum bevorzugt mit dem gasgefüllten Kopfraum des Bioreaktors verbindet. Eine andere Lösung, bei der der Kanal den Hohlraum im Inneren des Mischerschaftes mit einem Gasvolumen außerhalb des Beutels verbindet, ist ebenso denkbar, jedoch besteht hier die Gefahr einer Kontamination des Beutelinnenraums. Vorzugsweise weist der Kanal gegenüber dem Kopfraum des Beutels oder gegenüber dem Gasvolumen außerhalb des Beutels einen hydrophoben, für Gase durchlässigen, aber für Mikroorganismen und Zellen undurchlässigen Filter auf.

Ein bedeutender Vorteil der vorliegenden Erfindung besteht darin, dass der Mischerschaft stufenlos verstellbar ist. Der Mischerschaft kann somit für Bioreaktoren verschiedenster Größe verwendet werden. Außerdem stellt die Vermeidung mechanischer Kleinteile wie Kugelrasten, insbesondere solche aus Metall, einen Vorteil dar, wenn der Beutel mit Hilfe von Gammastrahlen sterilisiert werden soll, da jedes Metallteil Keime von der Strahlung abschirmen könnte.

Die Erfindung wird durch die nachstehenden Figuren und Ausführungsbeispiele näher erläutert.

Dabei zeigen
Figur 1 den Beutel mit Mischvorrichtung in Übersicht und
Figur 2 die Verbindung von zwei Schaftelementen im Detail.

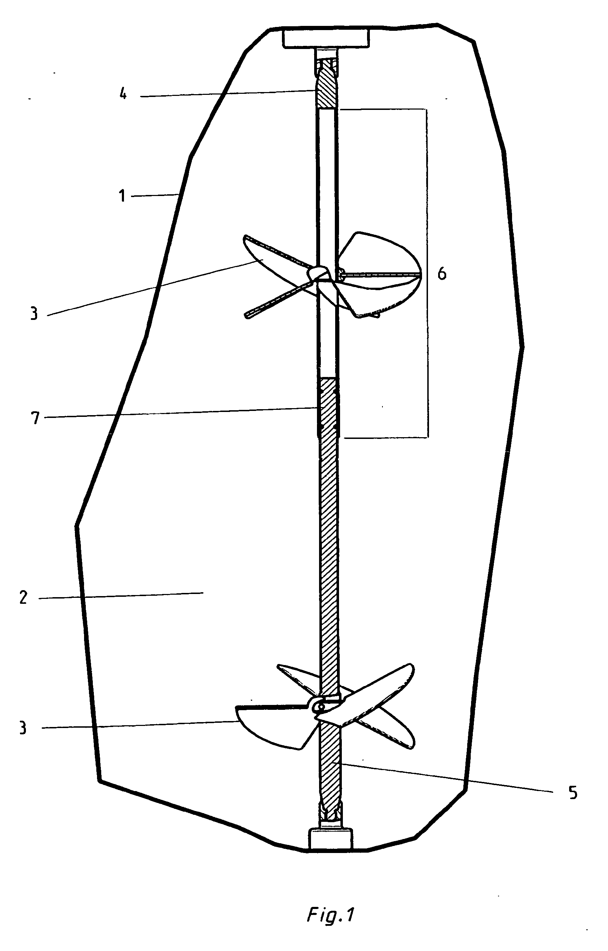

Gemäß Figur 1 befindet sich im Beutel 1 ein Innenraum 2, der einen Rührstab bestehend aus zwei Rührelementen 3 und einem Mischerschaft aufnimmt. Der Mischerschaft besteht aus einem ersten Schaftelement 4 und einem zweiten Schaftelement 5. Dabei weist das erste Schaftelement einen Hohlkörper 6 auf, indem ein Füllkörper 7 des zweiten Schaftelements verschiebbar gelagert ist.

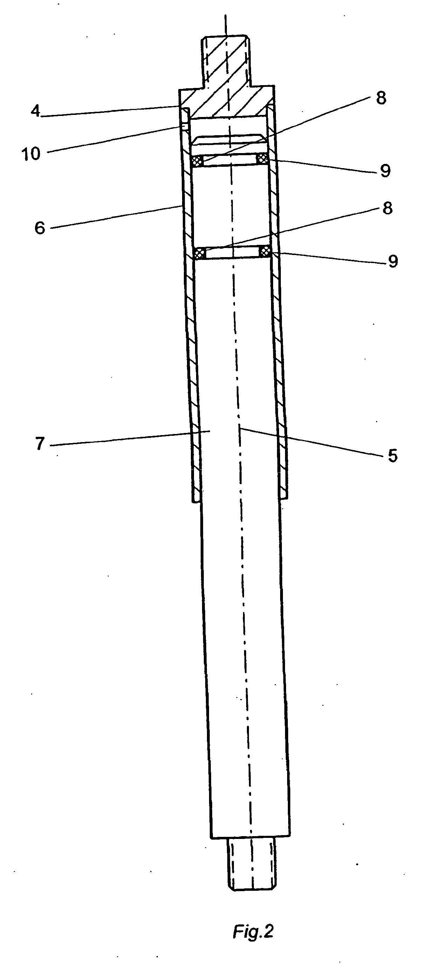

Gemäß Figur 2 sind in das erste Schaftelement 4 zwei Nuten 8 eingearbeitet, in denen jeweils ein Ring 9 eingelassen ist. Im Hohlkörper 6 des ersten Schaftelements 4 befindet sich ein Kanal 10, der den Hohlraum des Hohlkörpers mit dem Innenraum 2 des Beutels verbindet. Die Verbindung ist vorzugsweise mit einem hydrophoben, für Gase durchlässigen und für Mikroorganismen und Zellen undurchlässigen Filter, welcher über die Öffnung gesiegelt ist, abgedeckt (nicht dargestellt).

### Ausführungsbeispiel 1

Zwei O-Ringe mit einem Radius von 1 cm sollen ein Drehmoment von maximal 5 Nm übertragen. Die dafür notwendige Reibungskraft F_{R} zwischen den O-Ringen, dem Hohlkörper und dem Füllkörper ergibt sich aus dem maximalen Drehmoment Mₘ, und dem Umfang der O-Ringe U. U = 2rπ = 0,0628 m Fᵣ = Mₘ / U = 5 Nm / 0,0628 = 81 N

Dementsprechend ist eine Kraft von ca. 80 N ausreichend, um die Reibungskraft der O-Ringe zu überwinden und eine Längenverstellung des Mischerschaftes durchzuführen. Eine Kraft, die manuell oder durch die Hilfe einfacher mechanischer Vorrichtungen leicht aufgebracht werden kann.

## Patentansprüche

1. Flexibler Beutel (1) mit einer Mischvorrichtung, welche einen längenverstellbaren Mischerschaft, der in mindestens zwei oder mehrere Schaftelemente (4, 5) unterteilt ist, aufweist, wobei ein erstes von zwei benachbarten Schaftelementen (4) einen Hohlkörper (6) aufweist, in dem ein Füllkörper (7) eines zweiten Schaftelements (5) verschiebbar aufgenommen ist, **dadurch gekennzeichnet, dass** sich in dem Spalt zwischen dem Hohlkörper (6) des ersten Schaftelements (4) und dem Füllkörper (7) des zweiten Schaftelements (5) mindestens ein elastisches Element (9) befindet und ein Hohlraum im Innenraum des Mischerschaftes mit dem Innenraum (2) des Beutels (1) zum Druckausgleich durch einen Kanal (10) verbunden ist.

2. Flexibler Beutel nach Anspruch 1, **dadurch gekennzeichnet, dass** zumindest die beiden äußeren Schaftelemente an der der Beutelwand zugewandten Seite geschlossen sind.

3. Flexibler Beutel nach Anspruch 1, **dadurch gekennzeichnet, dass** der Hohlkörper (6) und der Füllkörper (7) der mindestens zwei Schaftelemente (4, 5) eine umlaufende, formschlüssige, ineinander greifende Kontur aufweisen.

4. Flexibler Beutel nach Anspruch 3, **dadurch gekennzeichnet, dass** der Hohlkörper (6) und der Füllkörper (7) der mindestens zwei Schaftelemente (4, 5) drehrund ausgebildet sind.

5. Flexibler Beutel nach Anspruch 3, **dadurch gekennzeichnet, dass** der Hohlkörper (6) und der Füllkörper (7) der mindestens zwei Schaftelemente (4, 5) vieleckig oder oval ausgebildet sind.

6. Flexibler Beutel nach Anspruch 1, **dadurch gekennzeichnet, dass** das mindestens eine elastische Element (9) teilweise in eine Nut (8) auf dem Füllkörper (7) des zweiten Schaftelements oder in eine Nut in dem Hohlkörper (6) des ersten Schaftelements (4) eingelassen ist.

7. Flexibler Beutel nach Anspruch 1, **dadurch gekennzeichnet, dass** das mindestens eine elastische Element (9) durch 2-K-Technologie an den Füllkörper (7) des zweiten Schaftelements (5) oder an den Hohlkörper (6) des ersten Schaftelements (4) angeformt ist.

8. Flexibler Beutel nach Anspruch 1, **dadurch gekennzeichnet, dass** das mindestens eine elastische Element (9) aus zwei beabstandeten Ringen besteht.

9. Flexibler Beutel nach einem der Ansprüche 1, 6, 7 oder 8, **dadurch gekennzeichnet, dass** durch das mindestens eine elastische Element (9) bei der Rotation des Mischerschaftes ein Drehmoment zwischen dem Hohlkörper (6) des ersten Schaftelements (4) und dem Füllkörper (7) des zweiten Schaftelements übertragbar ist.

10. Flexibler Beutel nach Anspruch 1, **dadurch gekennzeichnet, dass** durch das mindestens eine elastische Element (9) der Hohlraum im Inneren des Mischerschaftes gegen Flüssigkeiten aus dem Innenraum des Beutels (2) abgedichtet ist.

11. Flexibler Beutel nach Anspruch 1, **dadurch gekennzeichnet, dass** der Kanal (10) den Hohlraum im Inneren des Mischerschaftes und den mit Gas gefüllten Kopfraum des Beutels (1) zum Druckausgleich verbindet.

12. Flexibler Beutel nach Anspruch 1 oder 11, **dadurch gekennzeichnet, dass** der Kanal (10) gegen Flüssigkeiten durch eine für Gase durchlässige und für Mikroorganismen und Zellen undurchlässige hydrophobe Membran abgedichtet ist.

13. Flexibler Beutel nach einen der Ansprüche 6 bis 8, **dadurch gekennzeichnet, dass** der Hohlkörper (6) und/oder der Füllkörper (7) mindestens eine Nut aufweisen, in die eines der in einer Nut sitzenden oder durch 2-K-Technologie angeformten elastischen Elemente einrastbar ist.

## Claims

1. Flexible bag (1) with a mixing device comprising a mixer shaft which is adjustable in length and divided into at least two or more shaft elements (4, 5), wherein a first one of two adjacent shaft elements (4) comprises a hollow body (6) in which a filler body (7) of a second shaft element (5) is displaceably received, **characterised in that** at least one resilient element (9) is disposed in the gap between the hollow body (6) of the first shaft element (4) and the filler body (7) of the second shaft element (5) and a cavity in the interior space of the mixer shaft is connected by a channel (10) with the interior space (2) of the bag (1) for pressure equalisation.

2. Flexible bag according to claim 1, **characterised in that** at least the two outer shaft elements at the side facing the bag wall are closed.

3. Flexible bag according to claim 1, **characterised in that** the hollow body (6) and the filler body (7) of the at least two shaft elements (4, 5) have encircling, mechanically positively interengaging profiles.

4. Flexible bag according to claim 3, **characterised in that** the hollow body (6) and the filler body (7) of the at least two shaft elements (4, 5) are constructed to be round.

5. Flexible bag according to claim 3, **characterised in that** the hollow body (6) and the filler body (7) of the at least two shaft elements (4, 5) are constructed to be polygonal or oval.

6. Flexible bag according to claim 1, **characterised in that** the at least one resilient element (9) is partly let into a groove (8) on the filler body (7) of the second shaft element or into a groove in the hollow body (6) of the first shaft element (4).

7. Flexible bag according to claim 1, **characterised in that** the at least one resilient element (9) is formed at the filler body (7) of the second shaft element (5) or at the hollow body (6) of the first shaft element (4) by two-component technology.

8. Flexible bag according to claim 1, **characterised in that** the at least one resilient element (9) consists of two spaced-apart rings.

9. Flexible bag according to any one of claims 1, 6, 7 and 8, **characterised in that** a torque is transmissible between the hollow body (6) of the first shaft element (4) and the filler body (7) of the second shaft element by the at least one resilient element (9) on rotation of the mixer shaft.

10. Flexible bag according to claim 1, **characterised in that** the cavity in the interior of the mixer shaft is sealed by the resilient element (9) against liquids from the interior space of the bag (2).

11. Flexible bag according to claim 1, **characterised in that** the channel (10) connects the cavity in the interior of the mixer shaft and the head space, which is filled with gas, of the bag (1) for pressure equalisation.

12. Flexible bag according to claim 1 or 11, **characterised in that** the channel (10) is sealed against liquids by a hydrophobic membrane permeable by gases and impermeable by microorganisms and cells.

13. Flexible bag according to any one of claims 6 to 8, **characterised in that** the hollow body (6) and/or the filler body (7) has or have at least one groove in which one of the resilient elements seated in a groove or formed by two-component technology is detentable.

## Revendications

1. Sachet souple (1) comprenant un dispositif de mélange qui présente une tige de mélangeur pouvant être réglée en longueur, qui est subdivisée en au moins deux ou plusieurs éléments de tige (4, 5), où un premier parmi deux éléments de tige (4) voisins présente un corps creux (6), dans lequel un produit de remplissage (7) d'un second élément de tige (5) peut être reçu en coulissant, **caractérisé en ce que**, dans la fente entre le corps creux (6) du premier élément de tige (4) et le produit de remplissage (7) du second élément de tige (5), se trouve au moins un élément élastique (9) et qu'un corps creux à l'intérieur de la tige du mélangeur est relié avec la chambre intérieure (2) du sachet (1) par l'intermédiaire d'un canal (10) pour l'équilibre des pressions.

2. Sachet souple selon la revendication 1 **caractérisé en ce qu'**au moins les deux éléments de tige externes font corps avec la face orientée vers la paroi du sachet.

3. Sachet souple selon la revendication 1 **caractérisé en ce que** le corps creux (6) et le produit de remplissage (7) d'au moins deux éléments de tige (4, 5) présentent des contours sur toute la périphérie, complémentaires par la forme, imbriqués les uns dans les autres.

4. Sachet souple selon la revendication 3 **caractérisé en ce que** le corps creux (6) et le produit de remplissage (7) d'au moins deux éléments de tige (4, 5) sont conçus de manière circulaire.

5. Sachet souple selon la revendication 3 **caractérisé en ce que** le corps creux (6) et le produit de remplissage (7) d'au moins deux éléments de tige (4, 5) sont conçus de forme polygonale ou ovale.

6. Sachet souple selon la revendication 1 **caractérisé en ce qu'**au moins un élément élastique (9) a pénétré partiellement dans une rainure (8) sur le produit de remplissage (7) du deuxième élément de tige, ou dans une rainure dans le corps creux (6) du premier élément de tige (4).

7. Sachet souple selon la revendication 1 **caractérisé en ce qu'**au moins un élément élastique (9) est adapté par la forme sur le produit de remplissage (7) du deuxième élément de tige (5), ou sur le corps creux (6) du premier élément de tige (4), par une technologie 2-K.

8. Sachet souple selon la revendication 1 **caractérisé en ce qu'**au moins un élément élastique (9) est constitué de deux anneaux espacés.

9. Sachet souple selon l'une des revendications 1, 6, 7 ou 8 **caractérisé en ce qu'**un moment de rotation peut être transmis entre le corps creux (6) du premier élément de tige (4) et le produit de remplissage (7) du second élément de tige (5) par l'intermédiaire d'au moins un élément élastique (9) lors de la rotation de la tige du mélangeur.

10. Sachet souple selon la revendication 1 **caractérisé en ce que** le corps creux à l'intérieur de la tige du mélangeur est étanche vis-à-vis de liquides provenant de la chambre intérieure du sachet (2) par l'intermédiaire d'au moins un élément élastique (9).

11. Sachet flexible selon la revendication 1 **caractérisé en ce que** le canal (10) relie le corps creux à l'intérieur de la tige du mélangeur et la chambre en tête du sachet (1) remplie avec le gaz pour un équilibre des pressions.

12. Sachet souple selon les revendications 1 ou 11 **caractérisé en ce que** le canal (10) est rendu étanche vis-à-vis de liquides par une membrane hydrophobe perméable aux gaz et imperméable à des microorganismes et à des cellules.

13. Sachet souple selon l'une des revendications de 6 à 8 **caractérisé en ce que** le corps creux (6) et/ou le produit de remplissage (7) présentent au moins une rainure, dans laquelle un élément élastique, présent dans une rainure, ou adapté en forme par une technologie 2-K, peut être inséré.
